# EUROPEAN PATENT SPECIFICATION

(11) **EP 1 273 295 B1**
(45) Date of publication and mention of the grant of the patent: **27.08.2008**
(21) Application number: 00935232.9
(22) Date of filing: 09.06.2000
(51) Int. Cl.: A61K 31/327, A61K 31/385

(54) **COMPOSITION COMPRISING OZONIZED OILS AND/OR OTHER NATURAL AND/OR SYNTHETIC OZONIZED PRODUCTS AND USE THEREOF IN PHARMACEUTICAL, COSMETIC, DIETETIC OR FOOD SUPPLEMENT COMPOSITIONS, IN THE HUMAN AND VETERINARY FIELDS**
ZUSAMMENSETZUNG ENTHALTEND OZONISIERTE ÖLE UND/ODER ANDERE NATÜRLICHE UND/ODER SYNTHETISCHE OZONIZIERTE PRODUKTE UND IHRE VERWENDUNG IN PHARMAZEUTISCHEN, KOSMETISCHEN, DIÄTETISCHEN ODER NAHRUNGSERGÄNZENDEN ZUSAMMENSETZUNGEN IN HUMAN- UND VETERINÄRGEBIETEN
COMPOSITION COMPRENANT DES HUILES OZONISEES ET/OU AUTRES PRODUITS NATURELS ET/OU DE SYNTHESE OZONISES, ET SON UTILISATION DANS DES COMPOSITIONS PHARMACEUTIQUES, COSMETIQUES, DIETETIQUES OU DE COMPLEMENTS ALIMENTAIRES DANS LES DOMAINES HUMAIN ET VETERINAIRE

(30) Priority: 25.11.1999 ES 9902602
(43) Date of publication of application: 08.01.2003
(73) Proprietor: Gomez Moraleda, Manuel-Antonio, 08339 Vilassar De Dalt (ES); Dall'Aglio, Roberto, 08339 Vilassar De Dalt (ES); Melegari, Pierangelo, 08339 Vilassar De Dalt (ES)
(72) Inventor: Gomez Moraleda, Manuel-Antonio, 08339 Vilassar De Dalt (ES); Dall'Aglio, Roberto, 08339 Vilassar De Dalt (ES); Melegari, Pierangelo, 08339 Vilassar De Dalt (ES)
(86) International application number: PCT/ES2000/000208
(87) International publication number: WO 2001/037829

(56) References cited:
- EP-A1- 0 235 528
- EP-A2- 0 076 647
- DE-B- 1 255 660
- FR-A1- 2 730 636
- US-A- 4 451 480
- US-A- 5 904 924
- US-A- 5 948 443
- US-A- 5 977 162
- DATABASE WPI Week 199435, Derwent Publications Ltd., London, GB; AN 1994-284073D, XP002941525 & RU 2 008 898 C1 (SUKOLIN GA) 15 March 1994

## Description

### FIELD OF THE INVENTION

The present invention relates to compositions that comprise ozonized oils and/or natural and/or synthetic ozonized products, and thioctic acid and/or derivatives thereof, along with, optionally, other additional components. Said compositions are suitable for the production of pharmaceutical, cosmetic, dietary and food supplement compositions, in human beings and animals.

### BACKGROUND OF THE INVENTION

Ozonized oils and natural or synthetic ozonized products are natural or synthetic products that contain molecules with carbon-carbon (olefinic) double bonds, which, on being submitted to a reaction with ozone, in suitable fashion, produce a mixture of ozonides, oligomeric ozonides and different products rich in active oxygen, of the type of peroxide, hydroperoxide, etc. These substances are carriers of active oxygen and have a marked germicidal character, as well as possessing the capacity to stimulate different enzymatic processes of oxidation-reduction in cells, such as that of glutathione peroxidase, glutathione reductase, glucose-6-phosphatedehydrogenase, superoxide dismutase, catalase, etc., so consuming reduction equivalents such as NADH and NADPH. The aforementioned processes have a direct implication in the revitalisation of many vital and/or cell specific functions. For more than 30 years, these products have been used in limited fashion, as a way of application of ozone therapy, on the one hand by specialist in this field and in others, including the veterinary field. These products are of use in the local treatment in cases of infections, ulcers, crusts, burns and other tissue lesions, including the gastrointestinal tract. Similarly, some attempts have been made in the field of cosmetics for treatment of acne and for skin care, etc. There has also been observed evidence of systemic effects when administered by oral, parenteral or haematic way. Many of these effects are attributed to its capacity to stimulate different oxidation-reduction enzymatic processes in cells, such as that of glutathione peroxidase, glutathione reductase, glucose-6-phosphatedehydrogenase, superoxide dismutase, catalase, etc., which have a direct implication in many vital and/or cell specific functions.

Thioctic acid, also known as alpha-lipoic acid, alpha-lipoate, 1.2-dithiolan-3-pentanoic acid, etc, presents properties as a metabolic antioxidant, because many enzyme systems in animals treat it as a substrate for bio reductions. Supplemented thioctic acid is reduced to dihydrolipoate at the expense of cellular equivalents of reduction, such as NADH and NADPH. As the consumption of these reduction equivalents increases, the rate of cell metabolism also increases to make up for the increased demand. Thus, a property of thioctic acid is that of facilitating the use of the metabolic capacities of the cells themselves for recycling and enhancement. It is also considered capable of regenerating other natural antioxidants, such as vitamin C and E, glutathione, etc. Thioctic acid has been used in Europe for many years as an atoxic nutrient because of its capacity to help to maintain or restore hepatic health, as a treatment for several diseases related with toxins (mushroom poisoning, radiation, etc) and in alcoholic hepatitis. It has also been reported to be useful in patients with AIDS who receive antiviral or antibiotic treatments to contribute to the normalisation of the hepatic enzymes.

It has now been discovered, for the first time, that the association of ozonized oils and/or other natural and/or synthetic ozonized products with thioctic acid and/or derivatives thereof, along with, optionally, other substances of the type that will be mentioned later, acts as a perfect enhancer agent for the former, and said association is particularly advantageous in pharmaceutical, cosmetic, dietary and food supplement uses, in human and veterinary fields.

### DETAILED DESCRIPTION OF THE INVENTION

The invention provides a composition, hereinafter the composition of the invention, that comprises (i) one or more ozonized oils and/or one or more synthetic and/or natural ozonized products, and (ii) thioctic acid and/or derivatives thereof, in which each one of the components is found in a concentration lying between 0.01% and 99.99% by weight with respect to the total, preferably between 0.01% and 50% by weight.

The oils and natural or synthetic ozonized products can be obtained by means of the procedures described in the state of the art [Gabelein, K., "Therapeutische eigenschaften der aus ozongas gebildeten organische ozonide", Erfahr. hk. 23, 167-173 (1974); Gabelein, K., "Eine ganz besondere qualitat der ozonverbindungen", Erfahr. hk. 26, 203-209 (1977); German patent DE 12 55 660 (Gabelein, K., 1968); Japanese patent JP 55017351 (Rikagaku, K., 1980)].

In the sense used in this specification, the term "derivative" refers to substances constituted by the original molecule (in other words, the molecule from which they are derived), in which some of its constituent parts may be been substituted (chemical functional groups or atoms), by another chemical functional group, atom or molecule, in which the main structure is that of the original molecule. This term also includes the possibility that there has been performed the chemical addition of some other chemical functional group, atom or molecule to some of the bonds in the original structure.

The term "in any of its forms", as used in this specification in relation to a chemical element, means any substance more complex, either organic or inorganic, complex, etc., that contains it.

Similarly, as used in this specification, the term "conjugate" means substances constituted by the original molecule, or an essential part thereof, chemically combined with another of approximately the same size, or bigger, generally of the type of bases, such as those that constitute DNA, or others related thereto or derived there from. Specifically applied to melatonine, the term "conjugate" makes reference to any more complex form that contains melatonine or derivatives thereof and which serves to transport them to specific structures of certain cells.

The composition of the invention can contain, if so desired, in variable quantities, one or more active substances and/or additives and/or vehicles and/or excipients suitable for pharmaceutical, cosmetic, dietary or as food additive use, in human or veterinary fields.

In a particular embodiment, the composition of the invention contains one or more active substances that significantly enhance their effectiveness, selected from among, for example, substances that facilitate the stimulation of cellular biochemical processes, such as oxidation-reduction of oxidising and/or reducing metabolites, glycolysis, oxidative decarboxylation of pyruvic acid, the Krebs cycle, the cellular respiratory chain, etc., as well as substances that facilitate synthesis at cellular level of glutathione, NAD-NADH, NADP-NADPH, flavonoids, enzymes, co-factors, etc. Among said active substances there are found, for illustrative purposes that do not limit the scope of the present invention, among others:
- amino acids and derivatives thereof, for example, cysteine, arginine, glycine, N-acetyl-cysteine, etc., and derivatives thereof, at a concentration, in the event that they are present, lying between 0.01% and 25% by weight with respect to the total;
- organic acids and derivatives thereof, for example, fumaric acid, succinic acid, eicosapentanoic acid, hyaluronic acid, pantotenic acid, etc., and derivatives thereof, in a concentration, in the event that they are present, lying between 0.01% and 25% by weight with respect to the total;
- essential micro-elements and oligo-elements, for example, selenium, magnesium, manganese, zinc, copper, silver, etc., in any of their forms (salts, oxides, hydroxides, etc.), at a concentration, in the event that they are present, lying between 0.01% and 10% by weight with respect to the total;
- vitamins, and precursors and derivatives thereof, for example, vitamins from groups A, B, C, D, E and F, their precursors and derivatives, at a concentration in the event that they were present, lying between 0.01% and 25% with respect to the total;
- glutathione and its derivatives, acetylglucosamine and its derivatives, bioflavonoids and their derivates, coenzyme Q10 and its derivatives, melatonine and its derivatives and conjugates, mono-, sesqui-, di- o tri-terpenes and their derivatives, at a concentration in the event that they were present, lying between 0.01% and 10% with respect to the total;
- essential fatty acids, for example, linoleic acids and acids of the Ω-3 and Ω-6 type, at a concentration in the event that they were present, lying between 0.01% and 50% with respect to the total;
- phytoestrogens and their derivatives, alkaloids such as nicotine, johimbin, carnitine, galantoin, etc., their derivatives and precursors, at a concentration in the event that they were present, lying between 0.01% and 10% with respect to the total;
- film-forming compounds, such as compounds that form protective films of the skin, for example, polyfluorocarbons, such as FOMBLIN (a polyfluorocarbon that forms a protective film on the skin and allows the exchange of oxygen) and similar, at a concentration in the event that they were present, lying between 0.01% and 10% with respect to the total; and
- microorganisms and/or probiotic ferments such as lactobacillus LC1, rhamnosus GG, B. subtilis, etc., at a concentration in the event that they were present, lying between 1% and 20% with respect to the total.

The composition of the invention may contain one or more of the aforementioned active substances.

The composition of the invention is characterised by the association between (i) ozonized oils and/or other natural and/or synthetic ozonized products, and (ii) thioctic acid and/or derivatives thereof, optionally along with one or more of the active substances of the aforementioned type and in that said association presents an enhancing effect of the activities of said ozonized oils and natural or synthetic ozonized products, which is particularly advantageous in the uses of cosmetics, dietary products, as food supplements, and pharmaceutical products, for example, in the prevention and treatment of dermatitis, infections, acne, ulcers (including gastro - duodenal ulcers), scabs, burns and other tissue lesions, including the gastrointestinal tract, various cellular dysfunctions, energy metabolism disorders and disorders of the enzymatic systems protecting against oxidants, ageing, effects derived from free radicals, conditions related with oxidative stress and stress in general, and others.

In the sense used in this description the term "oxidative stress" comprises any type of physiological and/or pathological condition related to an increase in the levels of peroxides and/or free radicals in general, whether due to abnormal reduction in the activity of the enzymes responsible for their metabolization, and consequently the control of said levels, or due to the concurrence of different factors that may condition its elevation, whether they be environmental, dietary, toxic, due to ageing, diseases, etc. Among the conditions that deliver to oxidative stress can be mentioned by way of example: physical or mental stress, fatigue, ageing, toxic habits, hepatic insufficiency, viral diseases, inflammatory and ischaemic processes, different types of dysmetabolisms, neurological diseases, etc. The increase in levels of free radicals and other oxidants, above the levels necessary for defence of the organism, produce damage due to oxidation in healthy tissues, especially when said situation extends in time, and at times, may lead to multi-organ failure.

The association of compounds provided by this invention also act on the metabolism of carbohydrates, fatty acids, lipids, in the cellular respiratory chain, etc., activating to a greater extent the mitocondrial functionality, as well as other functionalities.

A noticeable synergistic effect has been observed resulting in the combination of compounds provided by the present invention, which leads to much more intense effects of each one of the components.

It has also been found, in addition, that the compositions of the invention are useful for the preparation of medicaments, cosmetics, dietary compositions, food supplements, etc., for human and/or veterinary use.

Therefore, the invention also provides a pharmaceutical composition that comprises a composition of the invention and one or more pharmaceutically acceptable excipients and/or vehicles. In a particular embodiment, the invention provides a pharmaceutical composition suitable for the prevention and treatment of cellular dysfunctions related to oxidative stress and to the poor functioning of cellular biochemical processes in general.

The cosmetic compositions provided by this invention comprise a composition of the invention along with one or more suitable vehicles and/or excipients.

Similarly, the dietary compositions and the nutritional supplements provided by this invention comprise a composition of the invention along with one or more additives and/or vehicles, matrices or acceptable supports.

The following examples serve to illustrate the present invention and should not be considered as limiting the scope thereof.

### EXAMPLE 1

### Obtaining ozonized olive oil

Ozonized olive oil can be obtained by directly ozonizing food-grade olive oil with ozone at room temperature.

1 kg of olive oil is placed in a sterile vertical glass column reactor, which possesses, in its bottom part, an entrance for gas via a sintered porous glass disk. Next, a flow of 3 l/h (litres/hour) of a mixture of ozone and oxygen from an ozone generator with approximately 60 mg of ozone/l is made to pass through the sintered glass disk. In order to obtain the ozone, medicinal oxygen is used, which, by means of a suitable pressure reducer, feeds an ozone generator by silent electrical discharge. By controlling the voltage of the feed to the ozone generator and the flow of gas, the desired concentration of ozone in the gaseous mixture can be established.

Ozonization is performed continually for several hours until it is observed that the mixture in the reactor begins to solidify. At this time, the ozonization is detained and the ozonized oil is extracted. The product obtained is washed with a saturated solution of sodium bicarbonate in bi-distilled water and dried with anhydrous sodium sulphate. Next, the acidity index of the product is determined, which should not be greater than 15. If it is greater than this value, the product is washed once more with a solution of sodium bicarbonate until reaching the desired acidity. When this has been achieved, the product is bottled in a sterile container, covered and labelled.

The final product obtained contains part of the initial unreacted olive oil, and a mixture of monomeric and oligomeric ozonides, peroxides, hydroperoxides, etc., all of them rich in active oxygen.

### EXAMPLE 2

### Capsules for gastroduodenal ulcers

### Capsules

Some capsules are prepared with the following composition:

| Component | Percentage by weight (%) |
|---|---|
| Micronized activated silica gel | 50 |
| Ozonized sunflower oil | 48 |
| Thioctic acid | 1.9 |
| Melatonine | 0.1 |

960 g of ozonized oil are heated to 40° C in a hot-water bath, and in this oil, 38 g of thioctic acid and 2 g of melatonine are dissolved. After this, the mixture is cooled to room temperature. Next, 1 kg of micronized activated silica gel is introduced into a high-speed mixer. This is then covered and the mixture agitated. Little by little, the previous mixture is added through a side entrance port, until there is total absorption thereof. The product is encapsulated in gastric capsules (of gelatine), these containing 1 g of product.

### Efficacy assay

The efficacy of the encapsulated product was evaluated in 10 patients with ages lying between 29 and 61 years, who had gastroduodenal ulcers of sizes lying between 0.5 and 2 cm diameter, and whose gastric cultures were positive for the presence of *Helicobacter pylori.*

The indicated treatment consisted of taking a capsule of the product on getting up, before eating, and another on going to bed, more than 2 hours after the last meal of the day, for the first 7 days, and taking 1 capsule of the product on going to bed, more than 2 hours after the last meal of the day, for 35 days.

An evaluation was performed after 21 days and another at the end of the treatment. The results obtained are shown in Table 1.

**Table 1**

| **Results of the efficacy assay (capsules)** | | | | | |
|---|---|---|---|---|---|
| **Patient** | **Age** | **Examination after 21 days** | | **Examination after 42 days** | |
| | | **Ulcer** | ***H.pylori*** | **Ulcer** | ***H.pylori*** |
| FR | 35 | + | - | - | - |
| AG | 56 | + | - | - | - |
| MB | 61 | - | - | - | - |
| RA | 59 | + | - | - | - |
| PD | 42 | + | + | - | - |
| GP | 37 | + | - | - | - |
| | | | | | |
| CI | 49 | + | - | - | - |
| MF | 29 | + | + | + | + |
| GT | 52 | + | - | - | - |
| MR | 47 | + | - | - | - |

Nine patients showed healing of the ulcers (-) and cultures negative (-) to the presence of *H. pylori* at the end of the treatment. Only one patient remained positive for *H. pylori* (+) and a gastric ulcer (+) present at the end of the treatment, although it is worth pointing out that their size had reduced by 50%. No complications or side effects were observed during the treatment.

### EXAMPLE 3

### Post-resurfacing epithelial repairing cream

### Cream

A post-resurfacing epithelial repairing cream is prepared with the following composition:

| | Component | Percentage by weight (%) |
|---|---|---|
| 1 | Ozonized L15 jojoba oil | 10 |
| 2 | Thioctic acid | 1 |
| 3 | Glutathione | 0.1 |
| 4 | Colloidal silver | 3 |
| 5 | Acemulgor LAM | 9 |
| 6 | Cetyl alcohol | 1.5 |
| 7 | Stearin | 1.5 |
| 8 | Cetiol 868 | 1 |
| 9 | Cetiol V | 2 |
| 10 | Isopropyl stearate | 0.2 |
| 11 | DC 345 | 2 |
| 12 | Avocado oil | 1 |
| 13 | Panalane | 3 |
| 14 | Jojoba oil | 2 |
| 15 | DC 200-350 | 2 |
| 16 | Carbopol Ultrez | 0.5 |
| 17 | Deionised water | 57 |
| 18 | 18 beta-glycolic acid | 0.5 |
| 19 | Vitamin E | 0.1 |
| 20 | Liquapar PE | 0.8 |
| 21 | EDTA Na2 | 0.1 |
| 22 | Glycerine | 1.5 |
| 23 | Controx KS | 0.1 |
| 24 | Perfume Dersit 0.1 | |

The product is prepared as a cosmetic cream, mixing the components in a turbo emulsifier. The different components (from the fourth to the 22^{nd}) are mixed with the usual procedure. The ozonized L15 jojoba oil is heated to 40°C in a hot-water bath, and the thioctic acid and glutathione are dissolved therein, cooling straight away. This mixture is added to the remaining components in the turbo-emulsor after the different "oil" phases. Once the emulsion has formed, this is rapidly cooled. Once cool, the cream is packaged in 50-ml plastic tubes.

### Efficacy assay

The efficacy of the product was evaluated in 10 subjects with ages ranging from 43 to 64 years. These subjects had been submitted to a laser facial resurfacing treatment. The cream was applied immediately after treatment and, two times a day for the following days.

The effects following resurfacing treatments are usually stinging, scab formations, reddening and inflammation. To attenuate these sequelae, moisturising cosmetic creams are used with anti-oxidant components, but, often, these sequelae last for 7 or 8 days and, in some cases, are complicated with superadded infections.

The evolution of sequelae was evaluated for the group of subjects studied by means of an evaluation scale of degree of intensity, ranging from 1 to 3+, at 24 hours and 3 days after treatment.

The results obtained are presented in Table 2.

**Table 2**

| **Results of the efficacy assay (cream)** | | | | | | | | | |
|---|---|---|---|---|---|---|---|---|---|
| **Subject** | **Age** | **24 H** | | | | **3 Days** | | | |
| | | **St** | **Sc** | **Red** | **Inf** | **St** | **Sc** | **Red** | **Inf** |
| AV | 46 | ++ | ++ | +++ | ++ | - | + | + | - |
| RM | 58 | + | ++ | ++ | ++ | - | - | - | - |
| GL | 43 | + | + | ++ | ++ | - | - | - | - |
| GP | 64 | ++ | ++ | +++ | +++ | - | - | + | - |
| | | | | | | | | | |
| RL | 56 | | ++ | ++ | ++ | - | - | - | - |
| CP | 46 | ++ | +++ | +++ | +++ | - | - | - | - |
| AU | 49 | + | ++ | + | + | - | - | - | - |
| MC | 47 | +++ | ++ | ++ | ++ | - | + | + | + |
| SO | 63 | ++ | + | + | ++ | - | - | - | - |
| AO | 52 | +++ | | +++ | ++ | - | - | - | - |

| | | | | | | | | | |
|---|---|---|---|---|---|---|---|---|---|
| [St: Stinging; Sc: Scabs; Red: Reddening; Inf: Inflammation] | | | | | | | | | |

The results showed that the sequelae of the resurfacing treatment were, in general, less severe, and that the evolution towards their disappearance was quicker than with the use of other creams. After 3 days, no subject had stinging, only two maintained a small scab, 3 had slight reddening, and only one presented very slight inflammation. In no case was there any infection.

### EXAMPLE 4

### Yoghurt enriched with ozonized vegetal oil and thioctic acid for the regulation of slow and incomplete digestion and intestinal upsets

Yoghurts were prepared with ozonized vegetal oil and thioctic acid, suitable for the regulation of slow and incomplete digestions and intestinal upsets. They had the following composition:

| Component | Percentage by weight (%) |
|---|---|
| Yoghurt, unflavoured and no added sugar | 99.5 |
| Ozonized L15T15 sunflower oil | 0.2 |
| Thioctic acid | 0.1 |
| Vitamin C | 0.1 |
| Glycine | 0.1 |

For the preparation of the yoghurt, to each 995 g of yoghurt there are added in sterile conditions 2 g of ozonized sunflower oil, 1 g of thioctic acid, 1 g of vitamin C and 1 g of glycine, mixing all in cold conditions. The mixture is cooled, and packaged in portions of 100 g and kept refrigerated (1-10°C).

### Efficacy assay

The efficacy of the product in a group of subjects, suffering frequently from slow and heavy digestion, as well as frequent gastrointestinal upsets, accompanied by episodes of diarrhoea, was evaluated.

The subjects were recommended to take 100 g of prepared yoghurt with not sugar after each meal for 35 days.

The frequency of slow and heavy digestions, as well as episodes of diarrhoea was evaluated with four levels: frequent (+++), not very frequent (++), few (+) and none (-), 21 days after beginning the study and at the end thereof. The results obtained are shown in Table 3.

**Table 3**

| **Results of the efficacy assay (yoghurt)** | | | | | |
|---|---|---|---|---|---|
| **Patient** | **Age** | **Examination after 21 days** | | **Examination after 35 days** | |
| | | **Slow digestion** | **Diarrhoe a** | **slow digestion** | **Diarrhoe a** |
| PT | 35 | ++ | + | - | - |
| AM | 56 | ++ | ++ | - | - |
| EL | 61 | +++ | +++ | + | + |
| DT | 59 | ++ | + | - | - |
| AF | 42 | + | ++ | - | - |
| MP | 37 | + | + | - | - |
| NJ | 49 | +++ | ++ | + | - |
| VB | 29 | ++ | + | - | - |
| FC | 52 | + | + | - | - |
| BR | 47 | ++ | ++ | - | - |

As can be seen, the product was effective, as most of the subjects resolved their gastrointestinal problems. Only 2 subjects maintained some infrequent episode of heavy digestion, and one of them, some infrequent episode of occasional diarrhoea.

### EXAMPLE 5

### Capsules of ozonized vegetal oil and thioctic acid for increasing the vitality and control of fat and weight

### Capsules

Capsules are prepared with the following composition:

| Component | Percentage by weight (%) |
|---|---|
| Micronized clay | 80 |
| Ozonized sunflower oil L15T15 | 16.5 |
| Thioctic acid | 0.4 |
| Lactobacilli LC1 | 3 |
| Selenium methionine | 0.1 |

165 g of ozonized oil are heated to 40° C in a hot-water bath, and the following ingredients mixed in the following order: 4 g of thioctic acid and 1 g of selenium methionine. Next, the mixture is cooled to room temperature and 30 g of lactobacilli LC1 added, mixing until obtaining a homogeneous dispersion. Next, 800 g of micronized clay are introduced in a high-speed mixer, the mixer covered and agitation thereof started. Little by little, the previous mixture is added through a side orifice until complete absorption of the mixture is attained. The product is encapsulated in gastric capsules (of gelatine), with 1 g of product.

### Efficacy assay

The efficacy of the product was evaluated in 20 subjects with ages ranging from 25 to 57 years, who showed frequent symptoms of physical exhaustion and who presented various degrees of overweight.

The following treatment was indicated: perform 6 hours of moderate physical exercise a week (fitness), observe a maintenance diet of 3 kcal, take a capsule of the product on getting up before eating anything and another on going to bed, at least 2 hours after the last food of the day, for the first 10 days, and 1 capsule of the product on going to bed, at least 2 hours after the last food of the day, during other 32 days.

An evaluation was performed before starting treatment, another after 21 days and another at the end of treatment, in which the weight and fat percentage by plicometry, and the sensation of physical exhaustion during the morning, evening and night, in degrees of intensity 1 and 3+ were measured. The results obtained are presented in Table 4.

As can be observed, all subjects lost corporal weight, and most notably, the amount of fat. According to the means, a subject with a weight of 79 kg at the beginning and 18.2 kg of fat (79 x 0.23) loses, in the study period, 3.2 kg of total weight and loses 4.2 kg of fat; the difference can represent an increase in muscle mass. Regarding the physical exhaustion, this disappeared in most of the subjects and, in those in which it was still manifest, was of much lower intensity and frequency.

**Table 4**

| **Results of the efficacy assay (capsules)** | | | | | | | | | | | | | | | | | |
|---|---|---|---|---|---|---|---|---|---|---|---|---|---|---|---|---|---|
| | | | **INITIAL EXAMINATION** | | | | | **EXAMINATION AFTER 21 DAYS** | | | | | **EXAMINATION AFTER 42 DAYS** | | | | |
| | | | **Physical exhaustion** | | | | | **Physical exhaustion** | | | | | **Physical exhaustion** | | | | |
| **SUB** | **SEX** | **AGE** | **M** | **E** | **N** | **Weight (Kg)** | **% Fat** | **M** | **E** | **N** | **Weight (Kg)** | **% Fat** | **M** | **E** | **N** | **Weight (Kg)** | **% Fat** |
| CM | M | 26 | X | X | XXX | 78.4 | 22 | 0 | X | X | 77.2 | 20 | 0 | 0 | 0 | 75.3 | 17 |
| AR | M | 42 | XXX | X | X | 84.6 | 26 | X | 0 | X | 82.7 | 22 | 0 | 0 | 0 | 81.4 | 20 |
| RB | F | 45 | XX | X | XX | 66.5 | 20 | X | 0 | 0 | 66.0 | 19 | 0 | 0 | 0 | 66.2 | 18 |
| GA | M | 35 | 0 | XX | XXX | 81.7 | 32 | 0 | X | X | 78.5 | 27 | 0 | 0 | 0 | 77.4 | 24 |
| PM | M | 38 | X | XXX | XX | 76.3 | 28 | 0 | X | X | 75.1 | 25 | 0 | 0 | 0 | 73.4 | 22 |
| | | | | | | | | | | | | | | | | | |
| AA | F | 29 | XX | X | XXX | 67.4 | 18 | X | 0 | XX | 66.1 | 17 | 0 | 0 | X | 66.7 | 16 |
| SN | F | 27 | 0 | XX | XXX | 69.7 | 17 | 0 | X | X | 69.5 | 17 | 0 | 0 | 0 | 68.0 | 15 |
| PS | F | 25 | XXX | 0 | X | 64.2 | 18 | X | 0 | X | 65.7 | 16 | 0 | 0 | 0 | 64.8 | 13 |
| RA | M | 38 | XX | 0 | XX | 94.5 | 24 | X | 0 | X | 92.3 | 21 | 0 | 0 | 0 | 89.3 | 18 |
| ET | M | 43 | 0 | X | XXX | 88.5 | 18 | 0 | 0 | X | 87.5 | 16 | 0 | 0 | 0 | 85.4 | 14 |
| CM | M | 41 | X | XX | 0 | 82.7 | 20 | 0 | X | 0 | 81.7 | 17 | 0 | 0 | 0 | 78.4 | 13 |
| FU | M | 57 | XXX | X | XXX | 99.4 | 26 | X | 0 | XX | 97.2 | 23 | 0 | 0 | X | 92.6 | 18 |
| MR | F | 27 | XX | 0 | X | 64.3 | 18 | X | 0 | 0 | 65.2 | 18 | X | 0 | 0 | 65.0 | 17 |
| RM | F | 45 | XXX | 0 | X | 56.9 | 16 | X | 0 | 0 | 57.3 | 16 | 0 | 0 | 0 | 57.6 | 16 |
| NB | F | 32 | 0 | XXX | 0 | 75.6 | 18 | 0 | X | 0 | 75.1 | 17 | 0 | X | 0 | 74.2 | 15 |
| TG | F | 33 | X | X | X | 69.8 | 17 | 0 | 0 | 0 | 69.3 | 16 | 0 | 0 | 0 | 68.3 | 15 |
| GB | M | 43 | 0 | X | XX | 87.2 | 23 | 0 | 0 | 0 | 84.6 | 19 | 0 | 0 | 0 | 81.3 | 14 |
| AZ | M | 36 | 0 | XXX | XXX | 104.3 | 32 | 0 | XX | XX | 99.8 | 27 | 0 | 0 | X | 94.9 | 22 |
| IC | F | 39 | XX | X | 0 | 77.8 | 28 | X | 0 | 0 | 76.3 | 25 | 0 | 0 | 0 | 79.1 | 22 |
| BO | F | 44 | XXX | XX | X | 89.7 | 39 | X | X | X | 85.6 | 33 | 0 | 0 | 0 | 81.8 | 28 |
| | Me | | | | | 79.0 | 23.0 | | | | 77.6 | 20.6 | | | | 75.8 | 17.9 |

| | | | | | | | | | | | | | | | | | |
|---|---|---|---|---|---|---|---|---|---|---|---|---|---|---|---|---|---|
| [SUB: Subject; M: Morning; E: Evening; N: Night; Me: Mean] | | | | | | | | | | | | | | | | | |

## Claims

1. A composition that comprises (i) one or more ozonized oils and/or natural and/or synthetic ozonized products, and (ii) thioctic acid and/or derivatives thereof, in which each one of the components is found at a concentration lying between 0.01% and 99.99% by weight with respect to the total.

2. A composition according to claim 1, in which each one of the components is found at a concentration lying between 0.01% and 50% by weight with respect to the total.

3. A composition according to any of the previous claims, which comprises, in addition, one or more suitable active substances and/or additives and/or vehicles and/or excipients for pharmaceutical, cosmetic, dietary, or food supplement use, in the human and veterinary fields.

4. A composition according to claim 3, which comprises, in addition, an active substance selected from an amino acid, a derivative of an amino acid and mixtures thereof, at a concentration lying between 0.01% and 25% by weight with respect to the total.

5. A composition according to claim 4, in which said active substance is selected from cysteine, arginine, glycine, N-acetyl-cysteine, derivatives thereof and mixtures thereof, at a concentration lying between 0.01% and 25% by weight with respect to the total.

6. A composition according to claim 3, which comprises, in addition, an active substance selected from an organic acid, a derivative of an organic acid and mixtures thereof, at a concentration lying between 0.01% and 25% by weight with respect to the total.

7. A composition according to claim 6, in which said active substance is selected from fumaric acid, succinic acid, eicosapentanoic acid, hyaluronic acid, pantotenic acid, derivatives thereof, and mixtures thereof, at a concentration lying between 0.01% and 25% by weight with respect to the total.

8. A composition according to claim 3, which comprises, in addition, an active substance selected from vitamins, precursors thereof, derivatives thereof and mixtures thereof, at a concentration lying between 0.01% and 25% by weight with respect to the total.

9. A composition according to claim 8, in which said active substance is selected from a vitamin of groups A, B, C, D, E, F, their precursors, derivatives thereof and mixtures thereof, at a concentration lying between 0.01% and 25% by weight with respect to the total.

10. A composition according to claim 3, which comprises, in addition, at least, an active substance selected from the group formed by glutathione and/or derivatives thereof, acetylglucosamine and/or derivatives thereof, bioflavonoids and/or derivatives thereof, coenzyme Q10 and/or derivatives thereof, and mixtures thereof, at a concentration lying between 0.01% and 10% by weight with respect to the total.

11. A composition according to claim 3, which comprises, in addition, at least, an active substance selected from melatonine and/or derivatives thereof, mono-, sesqui-, di- or tri-terpenes and/or derivatives thereof, at a concentration lying between 0.01% and 10% by weight with respect to the total.

12. A composition according to claim 3, which comprises, in addition, at least, an active substance selected from essential fatty acids and/or derivatives thereof, at a concentration lying between 0.01% and 50% by weight with respect to the total.

13. A composition according to claim 12, in which said active substance is selected from linoleic acid and acids of the type Ω-3, Ω-6, and mixtures thereof, at a concentration lying between 0.01% and 50% by weight with respect to the total.

14. A composition according to claim 3, which comprises, in addition, at least, an active substance selected from phytoestrogens and derivatives thereof, alkaloids, derivatives and precursors thereof, at a concentration lying between 0.01% and 10% by weight with respect to the total.

15. A composition according to claim 14, in which said active substance is selected from nicotine, johimbin, carnitine, galantoin, derivatives and precursors thereof, and mixtures thereof, at a concentration lying between 0.01% and 10% by weight with respect to the total.

16. A composition according to claim 3, which comprises, in addition, at least, an active substance selected from film-forming compounds, at a concentration lying between 0.01% and 10% by weight with respect to the total.

17. A composition according to claim 16, in which said active substance is selected from polyfluorocarbons forming films protecting the skin, at a concentration lying between 0.01% and 10% by weight with respect to the total.

18. A composition according to claim 3, which comprises, in addition, at least, an active substance selected from micro-organisms and/or probiotic ferments, at a concentration lying between 1% and 20% by weight with respect to the total.

19. A composition according to claim 18, in which said active substance is selected from lactobacillus LC1, rhamnosus GG, B. subtilis, and mixtures thereof, at a concentration lying between 1% and 20% by weight with respect to the total.

20. A pharmaceutical composition that comprises a composition according to any of claims 1 to 19 and one or more pharmaceutically acceptable excipients and/or vehicles.

21. A cosmetic composition that comprises a composition according to any of claims 1 to 19 and one or more acceptable excipients and/or vehicles.

22. A dietary composition that comprises a composition according to any of claims 1 to 19 and one or more acceptable additives and/or vehicles.

23. A nutritional supplement that comprises a composition according to any of claims 1 to 19 and one or more acceptable additives and/or vehicles.

24. Composition according to any of claims 1 to 19 for use as a medicament.

25. Composition according to claim 24 for the prevention and treatment of dermatitis, infections, acne, ulcers, including gastroduodenal ulcers, crusts, burns, tissue lesions, various cellular dysfunctions, energy metabolism disorders and disorders of enzymatic systems protecting against oxidants, ageing, effects derived from free radicals, conditions related to oxidative stress and to stress in general in humans and/or in animals.

26. Composition according to claim 24 for the improvement of the human nutrition and health and/or for such treatment in humans and/or animals.

27. Composition according to claim 24 for cosmetic personal care and cosmetic treatment.

28. Use of a composition according to any of claims 1 to 19 for the preparation of a medicament for the prevention and treatment of dermatitis, infections, acne, ulcers, including gastroduodenal ulcers, crusts, burns, tissue lesions, various cellular dysfunctions, energy metabolism disorders and disorders of enzymatic systems protecting against oxidants, ageing, effects derived from free radicals, conditions related to oxidative stress and to stress in general in humans and/or in animals.

29. Use of a composition according to any of claims 1 to 19 for the preparation of a cosmetic for personal care and cosmetic treatment and/or for such treatment in humans and/or animals.

30. Use of a composition according to any of claims 1 to 19 for the preparation of a dietary composition or food supplement for the improvement of the human nutrition and health and/or for such treatment in humans and/or animals.

31. Use of the composition according to any of the claims 1 to 19 as a cosmetic for personal care and in cosmetic treatments and/or for such treatments in humans and/or animals.

## Patentansprüche

1. Eine Verbindung, die (i) eine oder mehr ozonisierte Öle und/oder natürliche und/oder synthetische ozonisierte Produkte und (ii) Thioctsäure und/oder Derivate davon enthält, bei der jeder der Bestandteile in einer Konzentration zwischen 0,01 und 99,9 Gew.-% vorkommt.

2. Eine Verbindung nach Anspruch 1, bei der jeder der Bestandteile in einer Konzentration zwischen 0,01 und 50 Gew.-% vorkommt.

3. Eine Verbindung nach einem der vorhergehenden Ansprüche, die zusätzlich eine oder mehrere geeignete Wirkstoffe und/oder Zusatzstoffe und/oder Trägersubstanzen und/oder Arzneistoffträger für die pharmazeutische, kosmetische oder diätische Verwendung oder die Verwendung als Nahrungsergänzungsmittel im human- und veterinärmedizinischen Bereich enthält.

4. Eine Verbindung nach Anspruch 3, die zusätzlich einen Wirkstoff enthält, der eine aus einer Aminosäure, einem Derivat einer Aminosäure oder Gemischen davon ausgewählt wird, mit einer Konzentration zwischen 0,01 und 25 Gew.-%.

5. Eine Verbindung nach Anspruch 4, bei der der besagte Wirkstoff aus Cystein, Arginin, Glyzin, N-Acetylcistein, Derivaten und Gemischen davon ausgewählt wird, mit einer Konzentration zwischen 0,01 und 25 Gew.-%.

6. Eine Verbindung nach Anspruch 3, die zusätzlich einen Wirkstoff enthält, der aus einer organischen Säure, dem Derivat einer organischen Säure und Gemischen davon ausgewählt wird, mit einer Konzentration zwischen 0,01 und 25 Gew.-%.

7. Eine Verbindung nach Anspruch 6, bei der der besagte Wirkstoff aus Fumarsäure, Hyalaronsäure, Pantothensäure, Derivaten und Gemischen davon ausgewählt wird, mit einer Konzentration zwischen 0,01 und 25 Gew.-%.

8. Eine Verbindung nach Anspruch 3, die zusätzlich einen Wirkstoff enthält, der aus Vitaminen, deren Vorstufen (Provitaminen), Derivaten und Gemischen davon ausgewählt wird, mit einer Konzentration zwischen 0,01 und 25 Gew.-%.

9. Eine Verbindung nach Anspruch 8, bei der der besagte Wirkstoff aus Vitaminen der Gruppen A, B, C, D, E, F, deren Vorstufen (Provitamine), Derivaten und Gemischen davon ausgewählt wird, mit einer Konzentration zwischen 0,01 und 25 Gew.-%.

10. Eine Verbindung nach Anspruch 3, die zusätzlich mindestens einen Wirkstoff enthält, der aus der Gruppe ausgewählt wird, die aus Glutathion und/oder Derivaten davon, Acetylglucosamin und/oder Derivaten davon, Bioflavonoiden und/oder Derivaten davon und Coenzym Q10 und/oder Derivaten und Gemischen davon gebildet wird, mit einer Konzentration zwischen 0,01 und 10 Gew.-%.

11. Eine Verbindung nach Anspruch 3, die zusätzlich/mindestens einen Wirkstoff enthält, der aus Melatonin und/oder Derivaten davon, Mono-, Sesqui-, Di- oder Tri-Terpenen ausgewählt wird, mit einer Konzentration zwischen 0,01 und 10 Gew.-%.

12. Eine Verbindung nach Anspruch 3, die zusätzlich mindestens einen Wirkstoff enthält, der aus essenziellen Fettsäuren und/oder Derivaten davon ausgewählt wird, mit einer Konzentration zwischen 0,01 und 50 Gew.-%.

13. Eine Verbindung nach Anspruch 12, bei der der besagte Wirkstoff aus Linolsäure und Säuren vom Typ Ω-3, Ω-6 und Gemischen davon ausgewählt wird, mit einer Konzentration zwischen 0,01 und 50 Gew.-%.

14. Eine Verbindung nach Anspruch 3, die zusätzlich mindestens Wirkstoff enthält, der aus Phytoestrogenen und Derivaten davon, Alkaloiden, Derivaten und Vorläufern davon ausgewählt wird, mit einer Konzentration zwischen 0,01 und 10 Gew.-%.

15. Eine Verbindung nach Anspruch 14, bei der der besagte Wirkstoff aus Nikotin, Yohimbin, Carnitin, Derivaten und Vorläufern davon ausgewählt wird, mit einer Konzentration zwischen 0,01 und 10 Gew.-%.

16. Eine Verbindung nach Anspruch 3, die zusätzlich mindestens einen Wirkstoff enthält, der aus filmbildenden Gemischen ausgewählt wird, mit einer Konzentration zwischen 0,01 und 10 Gew.-%.

17. Eine Verbindung nach Anspruch 16, bei der der besagte Wirkstoff aus Polyfluorkarbonen, die einen Haut schützenden Film bilden, ausgewählt wird, mit einer Konzentration zwischen 0,01 und 10 Gew.-%.

18. Eine Verbindung nach Anspruch 3, die zusätzlich mindestens einen Wirkstoff enthält, der aus Mikroorganismen und/oder probiotischen Bakterien ausgewählt wird, mit einer Konzentration zwischen 1 und 20 Gew.-%.

19. Eine Verbindung nach Anspruch 18, bei der der besagte Wirkstoff aus Lactobacillus LCI, Lactobacillus rhamnosus GG, Bacillus subtilis und Gemischen davon ausgewählt wird, mit einer Konzentration zwischen 1 und 20 Gew.-%.

20. Eine pharmazeutische Verbindung, die eine Verbindung nach einem der Ansprüche 1 bis 19 und einen oder mehrere pharmazeutisch geeignete Arzneistoffträger und/oder Trägersubstanzen enthält.

21. Eine kosmetische Verbindung, die eine Verbindung nach einem der Ansprüche 1 bis 19 und einen oder mehrere geeignete Arzneistoffträger und/oder Trägersubstanzen enthält.

22. Eine diätische Verbindung, die eine Verbindung nach einem der Ansprüche 1 bis 19 und einen oder mehrere geeignete Zusatzstoffe und/oder Trägersubstanzen enthält.

23. Ein Nahrungsergänzungsmittel, das eine Verbindung nach einem der Ansprüche 1 bis 19 und einen oder mehrere geeignete Zusatzstoffe und/oder Trägersubstanzen enthält.

24. Verbindung nach einem der Ansprüche 1 bis 19 zur Verwendung als Medikament.

25. Verbindung nach Anspruch 24 für die Prävention und die Behandlung von Dermatitis, Infektionen, Akne, Geschwüren, inklusive gastroduodenalen Geschwüren, Wundschorf, Verbrennungen, Gewebeverletzungen, zahlreichen zellulären Störungen, Stoffwechselstörungen und Störungen des Enzymsystems, die vor Oxidantien, Alterung, Effekten, die durch freie Radikale ausgelöst werden und Erkrankungen die auf oxidativen Stress und Stress im Allgemeinen bei Menschen und/oder Tieren zurückgehen, schützt.

26. Verbindung nach Anspruch 24 zur Verbesserung der menschlichen Ernährung und Gesundheit und/oder zur entsprechenden Behandlung bei Menschen und/oder Tieren.

27. Verbindung nach Anspruch 24 für die individuelle kosmetische Pflege und die kosmetische Behandlung.

28. Verwendung einer Verbindung nach einem der Ansprüche 1 bis 19 für die Entwicklung eines Medikaments zur Prävention und Behandlung von Dermatitis, Infektionen, Akne, Geschwüren, inklusive gastroduodenalen Geschwüren, Wundschorf, Verbrennungen, Gewebeverletzungen, zahlreichen zellulären Störungen, Stoffwechselstörungen und Störungen des Enzymsystems, die vor Oxidantien, Alterung, Effekte, die durch freie Radikale ausgelöst werden und Erkrankungen, die auf oxidativen Stress und Stress im Allgemeinen bei Menschen und/oder Tieren zurückzuführen sind, schützt.

29. Verwendung einer Verbindung nach einem der Ansprüche 1 bis 19 für die Entwicklung eines Kosmetikums für die individuelle Pflege und/oder entsprechende Behandlungen bei Menschen und/oder Tieren.

30. Verwendung einer Verbindung nach einem der Ansprüche 1 bis 19 für die Entwicklung einer diätischen Verbindung oder eines Nahrungsergänzungsmittels für die Verbesserung der menschlichen Ernährung und Gesundheit und/oder für entsprechende Behandlungen bei Menschen und/oder Tieren.

31. Verwendung der Verbindung nach einem der Ansprüche 1 bis 19 als Kosmetikum für die individuelle Pflege und für kosmetische Behandlungen und/oder für entsprechende Behandlungen bei Menschen und/oder Tieren.

## Revendications

1. Une composition qui comprend (i) une ou plusieurs huiles ozonisées et/ou produits ozonisés naturels et/ou synthétiques, et (ii) de l'acide thioctique et/ou ses dérivés, dans laquelle la concentration de chacun des composants représente entre 0,01 % et 99,99 % du total.

2. Une composition conforme à la description 1, dans laquelle la concentration de chacun des composants représente entre 0,01 % et 99,99 % du total.

3. Une composition qui, selon l'une des descriptions précédentes, comprend, en outre, un(e) ou plusieurs substances actives et/ou additifs et/ou excipients à usage pharmaceutique, cosmétique, alimentaire ou de complément alimentaire, dans les domaines humain et vétérinaire.

4. Une composition conforme à la description 3, qui comprend, en plus, une substance active choisie parmi un acide aminé, un dérivé d'acide aminé et leurs mélanges, à une concentration comprise entre 0,01 % et 25 % en poids par rapport au total.

5. Une composition conforme à la description 4, dans laquelle ladite substance active est choisie parmi la cystéine, l'arginine, la glycine, la N-acétyl-cystéine et leurs dérivés, à une concentration comprise entre 0,01 % et 25 % en poids par rapport au total.

6. Une composition conforme à la description 3, qui comprend, en outre, une substance active choisie parmi un acide organique, un dérivé d'acide organique et de leurs mélanges, à une concentration comprise entre 0,01 % et 25 % en poids par rapport au total.

7. Une composition conforme à la description 6, dans laquelle ladite substance active est choisie parmi l'acide fumarique, l'acide succinique, l'acide eicosapentaénoïque, l'acide hyaluronique, l'acide pantothénique, leurs dérivés et mélanges, à une concentration comprise entre 0,01 % et 25 % en poids par rapport au total.

8. Une composition conforme à la description 3, qui comprend, en outre, une substance active choisie parmi les vitamines, leurs précurseurs, dérivés et mélanges, à une concentration comprise entre 0,01 % et 25 % en poids par rapport au total.

9. Une composition conforme à la description 8, dans laquelle ladite substance active est choisie parmi une vitamine des groupes A, B, C, D, E, F, leurs précurseurs, dérivés et mélanges, à une concentration comprise entre 0,01 % et 25 % en poids par rapport au total.

10. Une composition conforme à la description 3, qui comprend, en outre/au moins, une substance active sélectionnée parmi le groupe formé par le glutathion et/ou ses dérivés, l'acétylglucosamine et/ou ses dérivés, les bioflavonoïdes et/ou leurs dérivés, la coenzyme Q10 et/ou ses dérivés et mélanges, à une concentration comprise entre 0,01 % et 25 % en poids par rapport au total.

11. Une composition conforme à la description 3, qui comprend, en outre, au moins une substance active sélectionnée parmi la mélatonine et/ou ses dérivés, les mono-, sesqui-, diou tri-terpènes et/ou leurs dérivés, à une concentration comprise entre 0,01 % et 25 % en poids par rapport au total.

12. Une composition conforme à la description 3, qui comprend, en outre, au moins une substance active choisie parmi les acides gras essentiels et/ou leurs dérivés, à une concentration comprise entre 0,01 % et 50 % en poids par rapport au total.

13. Une composition conforme à la description 12, dans laquelle ladite substance active est choisie parmi l'acide linoléique et les acides du type Ω-3, Ω-6, et leurs mélanges, à une concentration comprise entre 0,01 % et 50 % en poids par rapport au total.

14. Une composition conforme à la description 3, qui comprend, en outre, au moins une substance active choisie parmi les phytoestrogènes et leurs dérivés, les alcaloïdes, leurs dérivés et précurseurs, à une concentration comprise entre 0,01 % et 10 % en poids par rapport au total.

15. Une composition conforme à la description 14, dans laquelle ladite substance active est choisie parmi la nicotine, la yohimbine, la carnitine, l'allantoïne, leurs dérivés et précurseurs, ainsi que leurs mélanges, à une concentration comprise entre 0,01 % et 10 % en poids par rapport au total.

16. Une composition conforme à la description 3, qui comprend, en outre, au moins une substance active choisie parmi les composés filmogènes, à une concentration comprise entre 0,01 % et 10 % en poids par rapport au total.

17. Une composition conforme à la description 16, dans laquelle ladite substance active est choisie parmi les polyfluorocarbones qui forment les films protecteurs de la peau, à une concentration comprise entre 0,01 % et 10 % en poids par rapport au total.

18. Une composition conforme à la description 3, qui comprend, en outre, au moins une substance active choisie parmi les micro-organismes et/ou les ferments probiotiques, à une concentration comprise entre 1 % et 20 % en poids par rapport au total.

19. Une composition conforme à la description 18, dans laquelle ladite substance active est choisie parmi le lactobacillus LCI, le rhamnosus GG, le B. subtilis et leurs mélanges, à une concentration comprise entre 1 % et 20 % en poids par rapport au total.

20. Une composition pharmaceutique qui comprend une composition conforme à l'une des descriptions 1 à 19 et un ou plusieurs additifs et/ou excipients acceptables.

21. Une composition cosmétique qui comprend une composition conforme à l'une des descriptions 1 à 19 et un ou plusieurs additifs et/ou excipients acceptables.

22. Une composition alimentaire qui comprend une composition conforme à l'une des descriptions 1 à 19 et un ou plusieurs additifs et/ou excipients acceptables.

23. Un complément nutritionnel qui comprend une composition conforme à l'une des descriptions 1 à 19 et un ou plusieurs additifs et/ou excipients acceptables.

24. Composition conforme à l'une des descriptions 1 à 19, à utiliser comme un médicament.

25. Composition conforme à la description 24 pour la prévention et le traitement de la dermatite, des infections, de l'acné, des ulcères (ulcères gastroduodénaux compris), des croûtes, brûlures, lésions de la peau, de divers dysfonctionnements cellulaires, troubles du métabolisme énergétique et troubles des systèmes enzymatiques de protection contre les oxydants, du vieillissement, des effets dérivés des radicaux libres, des états liés au stress oxydatif et au stress en général chez les hommes et/ou les animaux.

26. Composition conforme à la description 24 pour améliorer la nutrition et la santé de l'homme et/ou pour un tel traitement sur les hommes et/ou les animaux.

27. Composition conforme à la description 24 pour des soins cosmétiques personnels et un traitement cosmétique.

28. Utilisation d'une composition selon l'une des descriptions 1 à 19 pour la préparation d'un médicament pour la prévention et le traitement des dermatites, infections, de l'acné, des ulcères (ulcères gastroduodénaux compris), des croûtes, brûlures, lésions de la peau, divers dysfonctionnements cellulaires, troubles du métabolisme énergétique et troubles des systèmes enzymatiques de protection contre les oxydants, du vieillissement, des effets dérivés des radicaux libres, des états liés au stress oxydatif et au stress en général chez les hommes et/ou les animaux.

29. Utilisation d'une composition selon l'une des descriptions 1 à 19 pour la préparation d'un cosmétique pour des soins personnels et un traitement cosmétique et/ou pour un traitement de ce type sur les hommes et/ou les animaux.

30. Utilisation d'une composition selon l'une des descriptions 1 à 19 pour la préparation d'une composition alimentaire ou d'un complément alimentaire pour améliorer la nutrition et la santé de l'homme et/ou pour un tel traitement sur les hommes et/ou les animaux.

31. Utilisation de la composition selon l'une des descriptions 1 à 19 comme produit cosmétique pour des soins personnels ou dans des traitements cosmétiques et/ou pour ces traitements sur les hommes et/ou les animaux.
